# FASCICULE DE BREVET EUROPEEN

(11) **EP 3 083 547 B1**
(45) Date de publication et mention de la délivrance du brevet: **05.09.2018**
(21) Numéro de dépôt: 14812472.0
(22) Date de dépôt: 15.12.2014
(51) Int. Cl.: C07C 62/32

(54) **PROCEDE DE SEPARATION DE COMPOSES MANDELIQUES SOUS FORME SALIFIEE ET SON UTILISATION POUR LA PREPARATION D'ALDEHYDE AROMATIQUE**
VERFAHREN ZUR TRENNUNG VON MANDELSÄUREVERBINDUNGEN IN SALZFORM UND VERWENDUNG DAVON ZUR HERSTELLUNG VON AROMATISCHEM ALDEHYD
METHOD FOR SEPARATING MANDELIC COMPOUNDS IN SALIFIED FORM AND USE OF SAME FOR PREPARING AROMATIC ALDEHYDE

(30) Priorité: 18.12.2013 FR 1302986
(43) Date de publication de la demande: 26.10.2016
(73) Titulaire: Rhodia Operations, 75009 Paris (FR)
(72) Inventeur: GAREL, Laurent, F-69003 Lyon (FR); NORMAND, Stéphanie, F-69230 Saint-Genis-Laval (FR); FOUCHER, Stéphanie, Shanghai (CN); HORBEZ, Dominique, F-95130 Franconville (FR); ASSAM, Morad, F-69770 Chambost-Longessaigne (FR)
(74) Mandataire: Menville, Laure
(86) Numéro de dépôt international: PCT/EP2014/077701
(87) Numéro de publication internationale: WO 2015/091328

(56) Documents cités:
- WO-A1-2009/141280
- WO-A1-2011/039331

## Description

La présente invention se rapporte au domaine de la séparation de composé(s) aromatique(s) mandélique(s) et de la préparation d'aldéhyde(s) aromatique(s) à partir du(es)dit(s) composé(s) aromatique(s) mandélique(s).
Dans l'exposé qui suit de l'invention, le groupement mandélique s'entend du groupe - CHOH-COOH, lequel est présent en tant que substituant sur le noyau aromatique du(es)dit(s) composé(s) aromatique(s) mandélique(s).
La présente invention vise plus particulièrement la préparation de l'acide 4-hydroxy-3-méthoxybenzaldéhyde, encore appelé vanilline, à partir d'un sel de l'acide 4-hydroxy-3-méthoxymandélique et la préparation de l'acide 3-éthoxy-4-hydroxybenzaldéhyde, encore appelé éthylvanilline, à partir d'un sel de l'acide 3-éthoxy-4-hydroxymandélique.

La vanilline est obtenue à partir de sources naturelles telles que la lignine, l'acide férulique mais une part importante de la vanilline est produite par voie chimique. De nombreuses méthodes de préparation diverses et variées, sont décrites dans la littérature (KIRK-OTHMER - Encyclopedia of Chemical Technology 24, p 812-825, 4ème édition (1997)). Une voie d'accès classique à la vanilline implique une réaction de condensation d'acide glyoxylique sur le gaïacol en milieu basique, pour obtenir l'acide 4-hydroxy-3-méthoxymandélique. Le gaïacol est extrait de son milieu réactionnel avant d'être soumis à une étape d'oxydation. La vanilline est ensuite obtenue après neutralisation du sel de vanillate obtenu à l'issue de ladite étape d'oxydation et extraction subséquente de la vanilline. La demande WO2009/141280 décrit en particulier un procédé de séparation d'un composé mandélique sous forme salifiée, à partir d'un milieu réactionnel aqueux résultant de la réaction de l'acide glyoxylique en présence d'une base, comprenant une étape de mise en contact du milieu réactionnel avec une résine conduisant à l'adsoprtion sélective du composé phénolique de départ et à la récupération d'un flux aqueux comprenant les composés mandéliques sous forme salifiée. Ce procédé classique, bien que performant, présente les inconvénients d'une consommation énergétique, sous forme de vapeur, élevée, d'une génération importante d'effluents salins et d'une consommation élevée de base, généralement de soude, et d'acide minéral fort, généralement d'acide sulfurique. Pour diminuer à la fois les quantités de sels générés et les quantités de base et d'acide fort, une première amélioration a été apportée en procédant à la récupération des sels d'acides mandéliques, à l'issue de la réaction de condensation, par adsorption sélective de l'excès de gaïacol (WO 2011/039331). Toutefois, cette amélioration souffre de la nécessité d'opérer l'étape d'adsorption avec une concentration importante de gaïacol dans le flux à séparer et en conséquence de disposer d'un dispositif d'adsorption conséquent. Le besoin de soulager le dispositif d'adsorption pour espacer davantage les phases de régénération et traiter une quantité amoindrie de flux à séparer est grand et la présente invention a pour objectif de remédier aux inconvénients rencontrés lors de la récupération des sels d'acides mandéliques par simple adsorption.
Un autre objectif de la présente invention est d'améliorer la compétitivité du procédé de préparation d'aldéhyde aromatique, notamment de vanilline et d'éthylvanilline, en cherchant à réduire toujours plus la quantité de sels générés lors des différentes étapes de neutralisation. Cet objectif est atteint par la mise en place d'une technique alternative d'acidification du flux aqueux basique issu de l'étape d'oxydation, ledit flux étant acidifié par l'emploi de la technique d'électrodialyse, laquelle ne nécessite pas l'apport d'une source acide extérieure. Il en résulte une réduction drastique de la génération de sels, particulièrement de sels de sulfate, au niveau d'une section du procédé qui conduit, jusqu'à présent, à une forte production de sels.
Il résulte de la mise en place du procédé de l'invention, combinant à la fois la séparation de sels d'acides mandéliques par adsorption sélective précédée d'une étape de décantation et la neutralisation du flux basique d'oxydation par électrodialyse, une réduction considérable des sels produits par rapport à la quantité produite avec la mise en oeuvre du procédé actuel. Une diminution d'au moins 85% de sels produits, de préférence d'au moins 90% voire d'au moins 98% a été atteinte avec le procédé de préparation d'aldéhyde aromatique selon l'invention.

La présente invention a d'abord pour objet un procédé de séparation d'au moins un composé mandélique sous forme salifiée à partir d'un milieu réactionnel aqueux résultant de la réaction de condensation, dans l'eau, d'au moins un composé aromatique porteur d'au moins un groupe hydroxyle et dont la position en para est libre avec l'acide glyoxylique en milieu basique, ledit procédé comprenant au moins les étapes suivantes :
a) La décantation dudit milieu réactionnel de manière à récupérer une phase organique comprenant essentiellement de l'excès dudit composé aromatique et une phase aqueuse formée d'au moins dudit composé mandélique et d'une quantité de l'excès dudit composé aromatique,
b) La mise en contact de ladite phase aqueuse avec un support adsorbant conduisant à l'adsorption sélective dudit composé aromatique sur ledit support et à la récupération d'un flux aqueux comprenant au moins ledit composé mandélique sous forme salifiée.

Dans l'exposé qui suit, on entend par « composé aromatique » un composé cyclique présentant des doubles liaisons délocalisées telles que défini dans la littérature, notamment par M. SMITH et J. MARCH Advanced Organic Chemistry, 5ème Edition, John Wiley and Sons, 1992, pp.46 et suivantes.
Le procédé de séparation selon l'invention s'applique tout particulièrement à un composé aromatique tel que le phénol mais également aux phénols substitués ayant au moins une position en para- du groupe hydroxyle non substituée. Le noyau aromatique est porteur d'au moins un groupe hydroxyle mais il peut être également porteur d'un ou plusieurs autres substituants. Généralement, par plusieurs substituants, on définit moins de quatre substituants par noyau aromatique. N'importe quel substituant peut être présent dans la mesure où il n'interfère pas dans la réaction de condensation. Selon l'invention, ledit composé aromatique porteur d'au moins un groupe hydroxyle est aussi appelé « composé aromatique hydroxylé ».
Ainsi, le procédé de séparation selon l'invention est bien adapté pour s'appliquer aux composés aromatiques hydroxylés répondant à la formule suivante (I) : 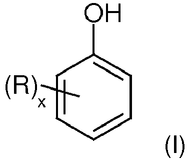 dans ladite formule,
- au moins la position en para du groupe hydroxyle est libre,
- R représente un atome d'hydrogène ou un ou plusieurs substituants, identiques ou différents,
- x, nombre de substituants sur un cycle, est un nombre inférieur ou égal à 4.

Dans la formule (I), les groupes R, identiques ou différents, représentent un atome d'hydrogène, un groupe alkyle, alcényle, alkoxy, hydroxyalkyle, alkoxyalkyle, cycloalkyle, aryle, arylalkyle, un groupe hydroxyle, un groupe nitro, un atome d'halogène, un groupe halogéno ou perhalogénoalkyle, un groupe formyle, un groupe acyle ayant de 2 à 6 atomes de carbone ; un groupe carboxylique, un groupe amino ou amido substitué ou non par un ou deux groupes alkyle ou phényle. Il est à noter que le groupe carboxylique peut être salifié de préférence par un métal alcalin (sodium, potassium) ou estérifié par exemple par un groupe alkyle ou phényle.

Dans la formule (I), lorsque x est supérieur à 1, deux groupes R placés sur deux atomes de carbone vicinaux, peuvent être liés entre eux par un groupe alkylène, alcénylène ou alcénylidène ayant de 3 à 5 atomes de carbone pour former un cycle saturé, insaturé ou aromatique ayant de 5 à 7 atomes : un ou plusieurs (de préférence 2 ou 3) atomes de carbone pouvant être remplacés par un hétéroatome, de préférence l'oxygène.
Dans le cadre de l'invention, on entend par « alkyle », une chaîne hydrocarbonée saturée linéaire ou ramifiée ayant de 1 à 15 atomes de carbone et de préférence de 1 ou 2 à 10 atomes de carbone.
Par « alkoxy », on entend un groupe alkyl-O- dans lequel le terme alkyle a la signification donnée ci-dessus. Des exemples préférés de groupes alkoxy sont les groupes méthoxy ou éthoxy.
Par « alcényle », on entend un groupe hydrocarboné, linéaire ou ramifié ayant de 2 à 15 atomes de carbone, comprenant une ou plusieurs doubles liaisons, de préférence, 1 à 2 doubles liaisons.
Par « cycloalkyle », on entend un groupe hydrocarboné cyclique, comprenant de 3 à 8 atomes de carbone, de préférence, un groupe cyclopentyle ou cyclohexyle.
Par « aryle », on entend un groupe mono- ou polycyclique aromatique, de préférence, mono- ou bicyclique comprenant de 6 à 12 atomes de carbone, de préférence, phényle ou naphtyle.
Par « arylalkyle », on entend un groupe hydrocarboné, linéaire ou ramifié porteur d'un cycle aromatique monocyclique et comprenant de 7 à 12 atomes de carbone, de préférence, benzyle.
Par « halogéno ou perhalogénoalkyle », on entend l'un des groupes suivants : -CX₃, -[CX₂]ₚ-CX₃ ou -CₚHₐF_{b} dans lesdits groupes, X représente un atome d'halogène, de préférence un atome de chlore ou de fluor ; p représente un nombre allant de 1 à 10, b un nombre allant de 3 à 21 et a + b = 2 p + 1.
Dans le cas où x est supérieur à 1, deux groupes R placés sur deux atomes de carbone vicinaux, peuvent être liés entre eux par un groupe alkylène, alcénylène ou alcénylidène pour former un cycle saturé, insaturé ou aromatique ayant de 5 à 7 atomes formant ainsi un bicycle. Des exemples de squelettes bicyliques préférés sont les suivants : 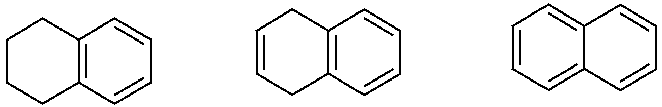 Les composés qui conviennent particulièrement bien à la mise en oeuvre du procédé de séparation de l'invention répondent à la formule (I) dans laquelle R, identiques ou différents, représentent :
- un atome d'hydrogène,
- un groupe hydroxyle,
- un groupe alkyle, linéaire ou ramifié, ayant de 1 à 6 atomes de carbone, de préférence de 1 à 4 atomes de carbone, tel que méthyle, éthyle, n-propyle, isopropyle, n-butyle, isobutyle, sec-butyle, tert-butyle,
- un groupe alcényle, linéaire ou ramifié, ayant de 2 à 6 atomes de carbone, de préférence de 2 à 4 atomes de carbone, tel que vinyle, allyle,
- un groupe alkoxy linéaire ou ramifié ayant de 1 à 6 atomes de carbone, de préférence de 1 à 4 atomes de carbone tel que les groupes méthoxy, éthoxy, n-propoxy, isopropoxy, n-butoxy, isobutoxy, sec-butoxy, tert-butoxy,
- un groupe phényle,
- un atome d'halogène, de préférence un atome de fluor, chlore ou brome.
Pour ce qui est de la définition de x, x est avantageusement égal à 0, 1 ou 2 et plus préférentiellement égal à 1.
De manière préférée, le procédé de l'invention s'applique aux composés répondant à la formule (I) dans laquelle R représente un atome d'hydrogène, un groupe alkyle ayant de 1 à 4 atomes de carbone et x est égal à 1 ou encore un groupe alkoxy ayant de 1 à 4 atomes de carbone et x est égal à 1.
A titre illustratif de composés répondant à la formule (I), on peut mentionner :
- ceux répondant à la formule (I) dans laquelle x est égal à 0, tels que :
   - le phénol,
- ceux répondant à la formule (I) dans laquelle x est égal à 1, tels que :
   - la pyrocatéchine
   - la résorcine
   - l'o-crésol
   - le m-crésol
   - le 2-éthylphénol
   - le 3-éthylphénol
   - le 2-propylphénol
   - le 2-sec-butylphénol
   - le 2-tert-butylphénol
   - le 3-tert-butylphénol
   - le 2-méthoxyphénol (gaïacol)
   - le 3-méthoxyphénol
   - le 2-éthoxyphénol (guétol)
   - le 2-isopropoxyphénol
   - l'aldéhyde salicylique
   - le salicylate de méthyle
   - le 2-chlorophénol
   - le 3-chlorophénol
   - le 3-nitrophénol
- ceux répondant à la formule (I) dans laquelle x est égal à 2, tels que :
   - le 2,3-diméthylphénol
   - le 2,5-diméthylphénol
   - le 3,5-diméthylphénol
   - le 2-hydroxy-5-acétamidobenzaldéhyde
   - le 2-hydroxy-5-éthamidobenzaldéhyde
   - le 2,3-dichlorophénol
   - le 2,5-dichlorophénol
   - le 3,5-dichlorophénol
   - le pyrogallol
- ceux répondant à la formule (I) dans laquelle x est égal à 3, tels que :
   - le 2,3,5-triméthylphénol
   - le 3,5-di-tertbutylphénol
   - le 2,3,5-trichlorophénol
- ceux répondant à la formule (I) présentant un groupe naphtalénique, tels que :
   - le 1-naphtol
   - le 2-naphtol
   - le 1,2-dihydroxynaphtalène
   - le 1,5-dihydroxynaphtalène
   - le 2,3-dihydroxynaphtalène
   - le 2,6-dihydroxynaphtalène
   - le 2,7-dihydroxynaphtalène
   - le 6-bromo-2-naphtol
- ceux répondant à la formule (I) présentant un enchaînement de noyaux benzéniques :
   - le 2-phénoxyphénol
   - le 3-phénoxyphénol
Parmi la liste des composés précités, les composés aromatiques porteurs d'au moins un groupe hydroxyle mis en oeuvre préférentiellement sont : le phénol, l'o-crésol, le m-crésol, le 3-éthylphénol, le 2-tert-butylphénol, le gaïacol, le guétol.
Les composés aromatiques hydroxylés auxquels s'applique préférentiellement le procédé de séparation de l'invention sont le gaïacol et le guétol, ou leur mélange.

Conformément au procédé de séparation de l'invention, ledit composé mandélique présent dans ledit milieu réactionnel résultant de ladite réaction de condensation est préférentiellement un composé p-hydroxymandélique, éventuellement substitué, dont la formule, sous forme acide, est représentée par la formule (II) suivante :

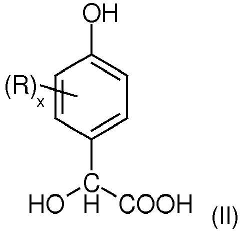

Dans ladite formule (II), R et x ont la signification donnée dans la formule (I).
Selon l'invention, ledit composé p-hydroxymandélique est présent dans ledit milieu réactionnel sous forme salifiée, c'est-à-dire que les atomes d'hydrogène du groupe hydroxyle et du groupe carboxylique COOH du composé mandélique représenté par la formule (II) sont remplacés par un cation métallique, en particulier un cation alcalin.

La réaction de condensation de laquelle résulte le mélange réactionnel soumis audit procédé de séparation selon l'invention est effectuée dans l'eau en présence d'au moins un composé aromatique hydroxylé et de l'acide glyoxylique, en phase liquide, en présence d'un agent alcalin. Comme agents alcalins, on fait appel de préférence à un hydroxyde de métal alcalin qui peut être notamment l'hydroxyde de sodium ou de potassium. Pour des considérations économiques, on choisit préférentiellement l'hydroxyde de sodium. La présence de la base conduit à la salification du composé aromatique hydroxylé d'une part et de la fonction carboxylique de l'acide glyoxylique d'autre part. La solution d'hydroxyde de métal alcalin mise en oeuvre a une concentration généralement comprise entre 10 et 50 % en poids. La quantité d'hydroxyde de métal alcalin introduite dans le milieu tient compte de la quantité nécessaire pour salifier la fonction hydroxyle du composé aromatique hydroxylé et la fonction carboxylique de l'acide glyoxylique. Généralement, la quantité d'hydroxyde de métal alcalin varie entre 70 et 120 % de la quantité stoechiométrique. L'acide glyoxylique peut être utilisé en solution aqueuse ayant une concentration variant par exemple, entre 15 et 99 % en poids ou peut être utilisé sous forme hydrate. On a recours, d'une manière préférée, aux solutions commerciales dont la concentration est d'environ 50% en poids.
On fait réagir l'acide glyoxylique sur au moins un composé aromatique hydroxylé. Le rapport molaire entre ledit composé aromatique hydroxylé et l'acide glyoxylique varie entre 1,0 et 4,0. Un mélange de plusieurs composés aromatiques hydroxylés peut être mis en oeuvre. De manière avantageuse, la réaction de condensation est effectuée en présence d'un catalyseur de type acide dicarboxylique comme décrit dans WO 99/65853. La quantité de catalyseur mise en oeuvre exprimée par le rapport entre le nombre de moles de catalyseur et le nombre de moles d'acide glyoxylique est choisie avantageusement entre 0,5 et 2,5, de préférence, entre 1 et 2. La température de la réaction de condensation est choisie avantageusement entre 0 et 100°C, et de préférence entre 10°C et 80°C. La réaction est conduite à pression atmosphérique, sous air ou sous atmosphère contrôlée de gaz inertes, de préférence d'azote ou éventuellement de gaz rares, en particulier l'argon. On choisit préférentiellement l'azote. La réaction de condensation peut être conduite dans différents types de réacteurs, par exemple dans un réacteur tubulaire (réacteur à écoulement piston) ou encore dans une cascade de réacteurs agités.

La réaction de condensation de laquelle résulte le mélange réactionnel soumis audit procédé de séparation selon l'invention est effectuée préférentiellement en mettant en oeuvre la réaction de l'acide glyoxylique avec le gaïacol ou la réaction de l'acide glyoxylique avec le guétol ou encore la réaction de l'acide glyoxylique avec le gaïacol et le guétol. Dans les deux premiers cas, la réaction de condensation conduit à la production d'un composé p-hydroxymandélique substitué en ortho du groupe hydroxyl respectivement par un groupe méthoxy (condensation de l'acide glyoxylique avec le gaïacol) et par un groupe éthoxy (condensation de l'acide glyoxylique avec le guétol), ledit composé p-hydroxymandélique étant sous forme salifiée. Dans le troisième cas, la réaction de condensation de l'acide glyoxylique avec le gaïacol et le guétol conduit à la co-production d'un composé p-hydroxymandélique salifié substitué en ortho du groupe hydroxyl par un groupe méthoxy et d'un composé p-hydroxymandélique salifié substitué en ortho du groupe hydroxyl par un groupe éthoxy.

Conformément au procédé de séparation de l'invention, ledit milieu réactionnel aqueux soumis à ladite étape a) de décantation comprend au moins ledit composé aromatique hydroxylé sous forme salifiée en excès et au moins un composé mandélique, préférentiellement un composé p-hydroxymandélique, sous forme salifiée. Ledit milieu réactionnel comprend également généralement d'autres composés mandéliques sous forme salifiée, en particulier un composé o-hydroxymandélique et un composé dimandélique hydroxylé (2 fonctions CHOH-COOM et une fonction OM, où M est un cation alcalin). La décantation dudit milieu réactionnel est facilitée car ledit milieu réactionnel est constitué d'une phase aqueuse et d'une phase organique qui ont une tendance à démixer. La phase organique est essentiellement formée de l'excès dudit composé aromatique hydroxylé sous forme non salifiée tandis que la phase aqueuse est principalement formée d'au moins dudit composé p-hydroxymandélique sous forme salifiée et d'une quantité de l'excès dudit composé aromatique hydroxylée sous forme salifiée. Ladite phase organique représente au plus 20% poids, de préférence au plus 10% poids, de manière encore plus préférée au plus 7% poids, dudit milieu réactionnel aqueux et elle est formée d'au moins 40% poids, de préférence d'au moins 70% poids dudit composé aromatique hydroxylé sous forme non salifiée. Ladite phase aqueuse est formée d'au moins 1% poids dudit composé p-hydroxymandélique et d'au plus 15% poids dudit composé aromatique hydroxylé sous forme salifiée.
Ledit milieu réactionnel est aqueux et basique. Le pH dudit milieu réactionnel, à l'issue de la réaction de condensation, est préférentiellement compris entre 10 et 12, généralement voisin de 11, c'est-à-dire compris entre 10,5 et 11,5.
Ladite étape de décantation est mise en oeuvre selon un fonctionnement en continu, semi-continu ou discontinu. De manière préférée, la décantation est mise en oeuvre selon un fonctionnement en continu.

Ladite étape de décantation est opérée à une température comprise entre 10 et 80°C. De manière préférée, elle est opérée à la température de la réaction de condensation. La durée de ladite étape de décantation est comprise de quelques minutes à quelques heures, généralement comprise entre 0,5 et 2 heures.

Selon un mode préféré de réalisation du procédé de l'invention, ladite phase organique obtenue après décantation est recyclée vers l'étape de réaction de condensation, en amont du procédé de séparation selon l'invention. La décantation de la phase organique dudit milieu réactionnel selon ladite étape a) du procédé de séparation de l'invention, en amont de ladite étape b) d'adsorption, permet de procéder au recyclage direct d'une quantité importante de l'excès dudit composé aromatique hydroxylé, présent dans ladite phase organique, sans avoir besoin de soumettre ladite quantité à l'étape d'adsorption subséquente. Il en résulte une diminution de la quantité de composé aromatique hydroxylé sous forme salifiée à adsorber sur ledit support adsorbant selon l'étape b) du procédé de séparation de l'invention et en conséquence un dimensionnement réduit de l'appareillage d'adsorption. La mise en oeuvre de la décantation permet de recycler directement de l'ordre de 20 à 80% poids, de préférence de 40 à 60% poids, de l'excès dudit composé aromatique hydroxylé vers l'étape de la réaction de condensation.

Conformément à ladite étape b) du procédé de séparation selon l'invention, ladite phase aqueuse, issue de ladite étape de décantation, est mise en contact avec un support adsorbant de manière à séparer ledit composé aromatique hydroxylé par adsorption. Plus précisément, ladite étape b) est effectuée par la mise en contact de la phase aqueuse issue de ladite étape a) et comprenant au moins un excès dudit composé aromatique hydroxylé et au moins ledit composé mandélique, préférentiellement au moins ledit composé p-hydroxymandélique, sous forme salifiée avec un support adsorbant conduisant à l'adsorption sélective dudit composé aromatique hydroxylé sur ledit support et à la récupération d'un flux aqueux comprenant au moins ledit composé p-hydroxymandélique. De manière préférée, ledit support adsorbant est choisi parmi les charbons actifs, les polymères adsorbants, les zéolithes, les tamis moléculaires et les résines échangeuses d'anions telles que décrites dans la demande de brevet WO 2009/141280. On choisit très préférentiellement un charbon actif à base de houille, de tourbe, de lignite, de résidus de distillation du pétrole, ou à partir de toute matière organique végétale riche en carbone: bois, écorces, brindilles, pâte de bois, coques de fruits de préférence coques de noix de coco, coques de cacahuètes. La quantité de support adsorbant mise en oeuvre est déterminée en fonction de l'efficacité d'adsorption du support adsorbant. L'Homme du métier saura adapter la quantité d'adsorbant pour avoir une adsorption complète. La quantité d'adsorbant représente au moins de 2 à 10 fois le poids du composé aromatique hydroxylé à adsorber. On met en oeuvre de préférence un excès d'adsorbant, par exemple un excès de 10 à 20 % du poids d'adsorbant calculé. De manière préférée, ladite étape b) est opérée à une température comprise entre 0 et 100°C, de préférence entre 15 et 80°C et de manière encore plus préférée à la température à laquelle ladite étape a) est opérée.
Les caractéristiques du support adsorbant et les avantages de la technique de séparation par adsorption / désorption sont explicités dans les demandes de brevet WO 2011/039331 et WO 2009/141280, en particulier les avantages résident en l'absence de nécessiter la mise en oeuvre d'une étape de neutralisation à la suite de l'étape de condensation : ledit composé aromatique hydroxylé sous forme salifiée est adsorbé sélectivement sur ledit adsorbant puis est récupéré avant d'être recyclé vers ladite étape de condensation, en amont du procédé de l'invention.
De manière avantageuse, ladite étape b) du procédé de séparation selon l'invention est mise en oeuvre en plaçant ledit support adsorbant dans un réacteur agité ou bien dans une colonne. Elle est opérée préférentiellement à co-courant : ladite phase aqueuse et ladite solution basique ont un sens de circulation identique, soit descendant soit ascendant, ce qui est favorable pour minimiser les quantités d'eau et de solution basique introduites pour la phase de régénération dudit composé aromatique hydroxylé.
Selon ladite étape b) du procédé de séparation selon l'invention, on récupère en sortie du dispositif d'adsorption, généralement une colonne, où se tient l'adsorption, un flux aqueux comprenant au moins un composé mandélique, préférentiellement au moins un composé p-hydroxymandélique, sous forme salifiée alors que le composé aromatique hydroxylé est adsorbé sur le support. De manière préférée, on récupère dans ledit flux aqueux non seulement ledit composé p-hydroxymandélique mais également un composé o-hydroxymandélique et un composé dimandélique hydroxylé.

L'excès dudit composé aromatique hydroxylé fixé sur l'adsorbant est désorbé par un traitement de régénération, en particulier par un traitement thermique, un traitement basique ou un traitement aqueux basique. Le traitement basique est assuré par l'emploi d'une base, par exemple la soude ou la potasse. Une solution aqueuse basique, ayant une concentration de 1 à 50 % en poids, est avantageusement employée. La soude est habituellement utilisée. La quantité de base mise en oeuvre est au moins égale à la quantité de composé aromatique hydroxylé à régénérer.
Selon un mode de réalisation préféré du procédé de séparation de l'invention, ledit composé aromatique hydroxylé, une fois désorbé, est recyclé sous forme salifiée vers l'étape de réaction de condensation, en amont du procédé de séparation selon l'invention. Un avantage réside en l'absence de la nécessité de procéder à la neutralisation dudit composé aromatique hydroxylé sous forme salifiée avant recyclage dès lors que ladite réaction de condensation met en oeuvre un composé aromatique hydroxylé sous forme salifiée.

Selon un autre mode de réalisation préféré du procédé de l'invention, il est avantageux de procéder à une étape d'acidification dudit milieu réactionnel préalablement à ladite étape de décantation et/ou à une étape d'acidification de ladite phase aqueuse, issue de ladite étape de décantation, préalablement à ladite étape de mise en contact avec un support adsorbant. De manière avantageuse, ladite étape d'acidification préalable à ladite étape de décantation est telle que la quantité de composé aromatique hydroxylé présent dans la phase aqueuse est abaissée de 10 à 70% poids par rapport à la quantité dudit composé aromatique présent dans ladite phase aqueuse lorsque le milieu réactionnel n'est pas soumis à une étape d'acidification. Ainsi, l'acidification préalable dudit milieu réactionnel permet d'augmenter sensiblement la quantité de composé aromatique hydroxylé directement recyclée vers l'étape de réaction de condensation et en conséquence d'opérer ladite étape b) d'adsorption dans des conditions optimisées dès lors qu'une quantité plus importante de la phase aqueuse, appauvrie en composé aromatique hydroxylé, peut être traitée avant de saturer le dispositif d'adsorption, généralement une colonne d'adsorption, et avant de procéder à la régénération. La mise en oeuvre de ladite étape d'acidification préalablement à ladite étape de décantation permet de recycler directement jusqu'à, voire au-delà, 80% poids de l'excès dudit composé aromatique hydroxylé vers l'étape de la réaction de condensation. Ladite étape d'acidification préalable à ladite étape de décantation peut être mise en oeuvre par toute méthode connue de l'Homme du métier, par exemple par emploi d'un acide fort, tel que l'acide sulfurique, un acide faible tel que l'acide acétique, l'acide formique ou le CO₂.

De manière très avantageuse, ladite étape d'acidification de ladite phase aqueuse, issue de ladite étape de décantation, préalablement à ladite étape de mise en contact avec un support adsorbant est telle que le pH est abaissé de 0,1 à 3 points. De manière préférée, le pH de ladite phase aqueuse est compris entre 8 et 11. L'acidification est effectuée par ajout d'acide fort ou d'acide faible ou encore par l'utilisation de CO₂. De manière avantageuse, on utilise l'acide sulfurique comme acide fort et l'acide formique, l'acide acétique ou le CO₂ comme acide faible. L'acidification de ladite phase aqueuse issue de ladite étape a) permet une meilleure adsorption dudit composé aromatique hydroxylé sous forme salifiée. Il est particulièrement avantageux de procéder à l'acidification de ladite phase aqueuse au moyen de l'acide formique ou de l'acide acétique ou encore au moyen de CO₂ de manière à ne pas générer la production de sels, notamment de sels sulfates.

Le CO₂, avantageusement employé à l'une et/ou l'autre desdites étapes d'acidification, provient préférentiellement de l'étape d) d'électro-électrodialyse ou d'électrodialyse à membrane bipolaire décrite ci-après dans la présente description qui génère la production de CO₂. On peut encore avantageusement utiliser du CO₂ recyclé à partir des effluents produits en aval du procédé de préparation d'aldéhyde hydroxyaromatique décrit ci-après dans la présente description.

Le procédé de séparation selon l'invention peut être mis en oeuvre en continu (au moyen de la technologie de lit mobile simulé ou « simulated moving bed » SMB), en semi-continu (carrousel de colonnes) ou en discontinu.

Le(s)dit(s) composé(s) mandélique(s) obtenu(s) à l'issue de ladite étape b) du procédé de séparation selon l'invention, préférentiellement ledit composé p-hydroxymandélique et généralement également un composé o-hydroxymandélique et un composé dimandélique hydroxylé, est(sont) particulièrement intéressant(s) car il s'agit de produits intermédiaires permettant entre autres, d'obtenir par réduction, des acides hydroxyarylacétiques ou par oxydation, des acides hydroxyarylglyoxyliques (= hydroxyaryl α-oxoacétiques) ou des aldéhydes hydroxyaromatiques.

Une application préférée du procédé de séparation selon l'invention est la préparation d'aldéhyde hydroxyaromatique, par oxydation de composé mandélique, en particulier de composé mandélique de formule (II) sous forme salifiée, obtenu à l'issue de ladite étape b) du procédé de séparation selon l'invention.

Un autre objet de la présente invention est un procédé de préparation d'aldéhyde hydroxyaromatique à partir de composé mandélique correspondant comprenant au moins les étapes suivantes :
c) Une réaction d'oxydation d'au moins dudit composé mandélique, obtenu selon le procédé de séparation décrit ci-dessus, en au moins un composé hydroxylate d'alkoxybenzaldéhyde,
d) La transformation d'au moins dudit composé hydroxylate d'alkoxybenzaldéhyde en aldéhyde hydroxyaromatique.

En particulier, l'invention concerne un procédé de préparation d'aldéhyde hydroxyaromatique à partir d'un milieu réactionnel aqueux résultant de la réaction de condensation, dans l'eau, d'au moins un composé aromatique porteur d'au moins un groupe hydroxyle et dont la position en para est libre avec l'acide glyoxylique en milieu basique, ledit procédé comprenant au moins les étapes suivantes :
a) La décantation dudit milieu réactionnel de manière à récupérer une phase organique comprenant essentiellement de l'excès dudit composé aromatique et une phase aqueuse formée d'au moins dudit composé mandélique et d'une quantité de l'excès dudit composé aromatique ;
b) La mise en contact de ladite phase aqueuse avec un support adsorbant conduisant à l'adsorption sélective dudit composé aromatique sur ledit support et à la récupération d'un flux aqueux comprenant au moins ledit composé mandélique sous forme salifiée ;
c) Une réaction d'oxydation d'au moins dudit composé mandélique en au moins un composé hydroxylate d'alkoxybenzaldéhyde ;
d) La transformation d'au moins dudit composé hydroxylate d'alkoxybenzaldéhyde en aldéhyde hydroxyaromatique.

L'invention concerne aussi un procédé de préparation d'aldéhyde hydroxyaromatique à partir d'au moins un composé aromatique porteur d'au moins un groupe hydroxyle et dont la position en para est libre, ledit procédé comprenant au moins les étapes suivantes :
- Une réaction de condensation, dans l'eau, dudit composé aromatique avec l'acide glyoxylique en milieu basique, et la récupération du milieu réactionnel aqueux résultant comprenant au moins un composé mandélique sous forme salifiée ;
- La décantation du milieu réactionnel de manière à récupérer une phase organique comprenant essentiellement l'excès dudit composé aromatique et une phase aqueuse formée d'au moins dudit composé mandélique et d'une quantité de l'excès dudit composé aromatique ;
- La mise en contact de ladite phase aqueuse avec un support adsorbant conduisant à l'adsorption sélective dudit composé aromatique sur ledit support et à la récupération d'un flux aqueux comprenant au moins ledit composé mandélique sous forme salifiée ;
- Une réaction d'oxydation d'au moins dudit composé mandélique en au moins un composé hydroxylate d'alkoxybenzaldéhyde ;
- La transformation d'au moins dudit composé hydroxylate d'alkoxybenzaldéhyde en aldéhyde hydroxyaromatique.

La réaction d'oxydation mise en oeuvre pour la conduite de ladite étape c) du procédé de préparation d'aldéhyde hydroxyaromatique selon l'invention est réalisée avantageusement en présence d'oxygène ou d'air. Ladite réaction d'oxydation est conduite à pression atmosphérique ou sous pression. Elle est préférentiellement opérée en milieu basique, généralement par ajout d'un agent alcalin ou alcalino-terreux. Ladite réaction d'oxydation est généralement mise en oeuvre en présence d'eau en tant que solvant. Elle est conduite à une température préférentiellement comprise entre 10 et 200°C. Ladite réaction d'oxydation est préférentiellement catalysée, soit par catalyse homogène soit par catalyse hétérogène. Un catalyseur de cette réaction d'oxydation peut être choisi parmi les catalyseurs comprenant au moins un élément métallique choisi parmi le groupe formé par le cuivre, le nickel, le cobalt, le fer, le manganèse, et l'un quelconque de leurs mélanges. Un mode de réalisation préféré de ladite réaction d'oxydation consiste à faire réagir au moins un composé mandélique sous forme salifiée, issu de ladite étape b) du procédé de séparation selon l'invention décrit ci-dessus, avec l'agent oxydant (oxygène ou air), en présence d'au moins un catalyseur d'oxydation. Ladite étape d'oxydation peut être conduite en continu ou en discontinu.

Le milieu réactionnel obtenu à l'issue de ladite réaction d'oxydation est un milieu aqueux basique dans lequel le contre-ion dudit hydroxylate d'alkoxybenzaldéhyde est préférentiellement un cation alcalin, préférentiellement le sodium ou le potassium, ou un cation alcalino-terreux. Il peut encore s'agir d'un cation ammonium.

Ladite réaction d'oxydation affecte la fonction mandélique sous forme salifiée, laquelle est transformée en une fonction aldéhyde. Le(s)dit(s) composé(s) mandélique(s), obtenu(s) selon le procédé de séparation décrit ci-dessus, est(sont) oxydé(s) en composé(s) hydroxylate d'alkoxybenzaldéhyde(s). Le terme oxydation s'entend ici comme une oxydation décarboxylative dans la mesure où elle comprend le départ d'un groupe carboxylate, formant par exemple du dioxyde de carbone.
De manière avantageuse, ledit composé mandélique sous forme salifiée, soumis à ladite réaction d'oxydation, est un sel de l'acide p-hydroxymandélique, un sel de l'acide 4-hydroxy-3-méthoxymandélique, un sel de l'acide 3-éthoxy-4-hydroxymandélique ou un sel de l'acide 4-hydroxy-3-isopropoxymandélique de manière à obtenir l'hydroxylate d'alkoxybenzaldéhyde correspondant. Il peut encore s'agir d'un mélange des sels des acides 4-hydroxy-3-méthoxymandélique et 3-éthoxy-4-hydroxymandélique pour obtenir, après oxydation, les hydroxylates d'alkoxybenzaldéhydes correspondants. Ledit sel de l'acide 4-hydroxy-3-méthoxymandélique correspond au sel de vanillate, précurseur de la vanilline, laquelle est alors obtenue à l'issue de la mise en oeuvre de ladite étape d) du procédé de préparation d'aldéhyde hydroxyaromatique selon l'invention, et ledit sel de l'acide 3-éthoxy-4-hydroxymandélique correspond au sel d'éthylvanillate, précurseur de l'éthylvanilline, laquelle est alors obtenue à l'issue de la mise en oeuvre de ladite étape d) du procédé de préparation d'aldéhyde hydroxyaromatique selon l'invention.

Conformément à ladite étape d) du procédé de préparation d'aldéhyde hydroxyaromatique selon l'invention, on procède à la transformation d'au moins dudit composé hydroxylate d'alkoxybenzaldéhyde en aldéhyde hydroxyaromatique.

Selon un mode préféré de réalisation du procédé de préparation d'aldéhyde hydroxyaromatique selon l'invention, ladite étape d) est mise en oeuvre par électrodialyse. Selon ledit mode, il s'agit de procéder à la neutralisation d'au moins dudit composé hydroxylate d'alkoxybenzaldéhyde en aldéhyde hydroxyaromatique au moyen de la technique d'électrodialyse. Ladite étape d) consiste en la transformation par neutralisation d'au moins dudit composé hydroxylate d'alkoxybenzaldéhyde en aldéhyde hydroxyaromatique et en la production d'une solution saline d'hydroxyde, préférentiellement d'une solution d'hydroxyde de sodium. De manière avantageuse, ladite étape d) est mise en oeuvre soit par électro-électrodialyse, encore appelée électrolyse à membrane, soit par électrodialyse à membrane bipolaire.

La mise en oeuvre par électro-électrodialyse est réalisée au moyen de membranes échangeuses de cations. La neutralisation d'au moins dudit composé hydroxylase d'alkoxybenzaldéhyde en aldéhyde hydroxyaromatique est opérée par la génération de protons H⁺ lesquels se substituent progressivement aux cations (généralement les ions Na⁺) présents dans le milieu réactionnel aqueux issu de ladite réaction d'oxydation, lesdits cations migrant, sous l'effet d'un champ électrique, en direction de la cathode, à travers une membrane échangeuse de cations (appelée membrane cationique) pour se combiner aux ions OH⁻ produits par réduction cathodique de l'eau et générant ainsi une solution d'hydroxyde de cations, préférentiellement une solution d'hydroxyde de sodium. Les membranes échangeuses de cations (MEC) comportent des groupements acides forts, préférentiellement des groupements sulfonates, ou des groupements acides faibles, préférentiellement des groupements carboxylates.
Les membranes cationiques employées pour la mise en oeuvre de la technique d'électrolyse à membrane sont choisies parmi les membranes hétérogènes et les membranes homogènes. Les membranes hétérogènes sont préparées à partir de particules de résines échangeuses de cations incorporées dans une résine phénolique ou un polymère (polychlorure de vinyle, polyéthylène ou autre). L'ensemble ainsi formé peut enduire une trame comme par exemple un tissu de polyester ou de polyacrylonitrile. Comme membranes cationiques hétérogènes avantageusement employées pour la mise en oeuvre de la technique d'électrolyse à membrane, on peut citer les membranes RALEX commercialisées par la société MEGA.
Les membranes homogènes sont obtenues par introduction d'un groupement fonctionnel sur un support inerte, par greffage chimique ou radiochimique. La méthode chimique, la plus utilisée, consiste généralement à fonctionnaliser un latex d'un polymère comportant des noyaux aromatiques, tel que styrène/divinylbenzène ou styrène/butadiène. Le latex ainsi fonctionnalisé sert ensuite à enduire une trame comme pour les membranes hétérogènes. Comme membranes cationiques homogènes avantageusement employées pour la mise en oeuvre de la technique d'électrolyse à membrane, on peut citer les membranes SELEMION commercialisées par ASAHI GLASS ou les membranes NEOSEPTA commercialisées par ASTOM.

De manière avantageuse et selon la technique d'électro-électrodialyse, ladite étape d) consiste en la transformation d'au moins dudit composé hydroxylate d'alkoxybenzaldéhyde en aldéhyde hydroxyaromatique et en la production d'une solution d'hydroxyde, préférentiellement d'une solution d'hydroxyde de sodium. Ladite étape d), mise en oeuvre par la technique d'électro-électrodialyse, est opérée au moyen d'un électrolyseur à deux compartiments. La figure 1 est une représentation schématique du fonctionnement d'un tel électrolyseur dans le cas où il s'agit d'opérer la transformation de vanillate de sodium (VA(Na)) en vanilline (VA). Il va de soi que cette représentation est un exemple permettant de faciliter la compréhension du fonctionnement de la technique d'électro-électrodialyse, le vanillate représentant ledit composé hydroxylate d'alkoxybenzaldéhyde et le sodium représentant lesdits cations présents dans le milieu réactionnel aqueux issu de ladite réaction d'oxydation.
Selon la technique d'électro-électrodialyse, les ions H⁺ et OH⁻ sont générés par les électrodes. En particulier, selon l'emploi d'un électrolyseur à deux compartiments, le milieu réactionnel aqueux à traiter, issu de ladite étape de réaction d'oxydation, est introduit dans le compartiment « sel » où se trouve l'anode sur laquelle va se produire l'oxydation de l'eau, selon la réaction : H₂O → 2 H⁺ + ½ O₂ + 2 e⁻.
Les ions Na+ migrent en direction de la cathode à travers une membrane échangeuse de cations et se combinent avec les ions OH⁻ produits par réduction cathodique de l'eau, selon la réaction : H₂O + e⁻ → OH⁻ + ½ H₂.
Dans une variante, on peut utiliser à l'anode une électrode à diffusion de gaz pour oxyder l'hydrogène généré selon la réaction : H₂ → 2H⁺ + 2 e⁻.
Dans une autre variante, on peut utiliser à la cathode une électrode à diffusion de gaz pour réduire de l'oxygène selon la réaction % O₂ + H₂O + e⁻ → 2 OH⁻.

Il est encore avantageux, pour protéger l'anode de la présence de composés organiques susceptibles de l'encrasser ou de s'y oxyder, d'opérer ladite étape d) au moyen d'un électrolyseur à trois compartiments. Dans cette configuration, on fait circuler dans le compartiment anodique un acide dont l'anion n'est pas oxydable, par exemple l'acide sulfurique. La figure 2 est une représentation schématique du fonctionnement d'un tel électrolyseur à trois compartiments. L'abréviation MEC sur la figure 2 signifie « membrane échangeuse de cations ».

Quel que soit le type de configuration de l'électrolyseur (deux, trois ou davantage de compartiments), les ions H⁺ et OH⁻ sont générés par les électrodes.

La température à laquelle est mise en oeuvre ladite étape d) lorsqu'elle est opérée par électro-électrodialyse se situe dans le domaine compatible avec les membranes cationiques. De manière avantageuse, la température est comprise entre 15 et 90°C. La densité de courant appliquée à l'électrolyseur est préférentiellement comprise entre 1000 et 3000 A/m².

La mise en oeuvre de ladite étape d) par électrodialyse à membrane bipolaire repose sur la génération des ions H⁺ et OH⁻, non pas par les électrodes, mais au moyen de ladite membrane bipolaire (MB) elle-même. Une membrane bipolaire est constituée de trois couches : une face échangeuse de cations, une face échangeuse d'anions et une interface hydrophile de jonction. Sous l'effet d'un champ électrique, l'eau de solvatation à l'interface de la membrane se dissocie en ions H⁺ et OH⁻, lesquels migrent respectivement vers la cathode en traversant la face cationique, et vers l'anode, en traversant la face anionique. Comme membranes bipolaires avantageusement employées pour la mise en oeuvre de la neutralisation par électrodialyse, on peut citer à titre d'exemple celle décrite dans la demande de brevet WO 96/01286 ou les membranes commercialisées sous le nom Neosepta® par ASTOM ou sous le nom fumasep® par FUMATECH. On peut encore avantageusement utiliser les membranes bipolaires commercialisées par la société MEGA.

Dans une configuration à deux compartiments, on introduit la solution de vanillate de sodium dans le compartiment « sel/acide ». Sous l'action du champ électrique, les ions Na+ migrent vers la cathode à travers une membrane échangeuse de cations et se combinent avec les ions OH⁻ provenant de la face anionique de la membrane bipolaire, pour former de l'hydroxyde de sodium dans le compartiment base. Simultanément, les ions H⁺ sont produits sur la face cationique de la membrane bipolaire, conduisant à une acidification (neutralisation) de la solution de vanillate.

Dans cette configuration (figure 3), un compartiment « sel/acide » et un compartiment base forment une cellule unitaire d'électrodialyse. Un électrodialyseur comporte un empilement de plusieurs cellules unitaires, dont le nombre peut varier avantageusement entre 5 et 300.

Outre les membranes, l'électrodialyseur à membranes bipolaires comporte à ses extrémités une anode et une cathode qui sont par exemple composées de titane revêtu d'un revêtement électrocatalytique, d'inox 316 L, de nickel. Au voisinage des électrodes, on fait circuler une solution d'électrolyte destinée à assurer une conductivité électrique suffisante : un anolyte à l'anode ; un catholyte à la cathode. On utilise souvent une solution unique d'électrolyte. L'électrolyte mis en oeuvre peut être un sel, un acide ou une base, choisi parmi les composés non électro-actifs. A titre d'exemple, on peut citer des sels neutres, comme les sulfates, des acides comme l'acide sulfurique ou des bases comme la soude.
La densité de courant appliquée à l'électrodialyseur à membranes bipolaires est généralement comprise entre 0.2 et 1.5 kA/m², de préférence entre 0.5 et 1.2 kA/m².
La température à laquelle est mise en oeuvre ladite étape d) lorsqu'elle est opérée par électrodialyse à membrane bipolaire se situe dans un domaine compatible avec la stabilité des membranes. De manière avantageuse, ladite étape d) est opérée à une température comprise entre 15 et 90°C. Selon un mode de réalisation particulier, ladite étape d) est opérée à une température comprise entre 45 et 90°C, de préférence entre 50 et 80°C, de manière encore plus préférée entre 50 et 70°C. Toute membrane bipolaire résistant à cette température convient. Les membranes bipolaires NEOSPEPTA®-BP1 et NEOSPEPTA®-HT sont avantageusement employées pour la mise en oeuvre de ladite étape d). Il est avantageux d'opérer à une température au-delà de 45°C car, outre le gain sur la consommation électrique du fait de l'augmentation de la conductivité électrique, le fonctionnement dans cette gamme de température plus élevée permet de mettre en oeuvre des solutions plus concentrées d'hydroxylate d'alkoxybenzaldéhyde, notamment de vanillate de sodium (la solubilité de la vanilline augmentant fortement avec la température).
La durée de mise en oeuvre de ladite étape d) conduite par électrodialyse à membrane bipolaire est préférentiellement inférieure à une heure.

L'électrolyseur ou l'électrodialyseur mis en oeuvre pour opérer ladite étape d) peut fonctionner de différentes façons. Il peut tout d'abord fonctionner en continu en passage direct ; plusieurs étages sont alors disposés en série pour atteindre le taux de conversion recherché. Il peut fonctionner en continu avec recirculation (mode « feed & bleed » ou mode « alimentation et soutirage »); enfin, il peut fonctionner en discontinu ; la solution recirculant jusqu'à obtention du taux de conversion ou du pH souhaité.
Pour obtenir un bon fonctionnement de l'électrolyseur ou de l'électrodialyseur, la conductivité électrique des solutions doit être suffisante. La conductivité du compartiment sel/acide diminuant au fur et à mesure de la conversion, il peut être avantageux d'adapter la densité de courant au taux de conversion, et de réaliser la conversion en deux voire trois étages fonctionnant à des densités de courant décroissantes. En fonctionnement multiétagé, les différents étages peuvent fonctionner selon le même mode ou selon des modes différents. Par exemple, lorsque l'étape d) est opérée en deux étages, plusieurs modes de réalisation sont possibles : les deux étages peuvent fonctionner en continu, les deux étages peuvent fonctionner en discontinu, le premier étage peut fonctionner en continu tandis que le deuxième étage peut fonctionner en discontinu, ou bien le premier étage peut fonctionner en discontinu tandis que le deuxième étage peut fonctionner en continu.

En outre, pour que le fonctionnement de l'électrolyseur ou de l'électrodialyseur soit stable et performant, un prétraitement des solutions mises en oeuvre est possible. Un prétraitement permet avantageusement de prévenir l'encrassement des membranes. L'homme du métier peut notamment choisir un prétraitement parmi les techniques connues telles que la filtration, l'ultrafiltration, le traitement sur charbon actif et le passage à travers des résines chélatantes. Ces dernières, particulièrement préférées, permettent de retirer sélectivement les ions inorganiques multivalents, en particuliers les ions Ca²⁺, Mg²⁺, Ba²⁺, Mn²⁺, Zn²⁺, Fe³⁺ et Sr²⁺. Elles permettent avantageusement aussi de fixer des résidus solubles du catalyseur qui pourrait éventuellement être utilisé lors de l'étape d'oxydation.

L'acidification par électrodialyse, mise en oeuvre par électro-électro-dialyse ou électrodialyse à membrane bipolaire, s'effectue jusqu'à une valeur de pH permettant le taux de conversion de l'hydroxylate d'alkoxybenzaldéhyde, notamment du vanillate en vanilline, recherché. Par exemple, dans le cas préféré où on recherche à produire de la vanilline, le pkA de la fonction phénol de la vanilline étant de 7,50 à 25°C, on choisira une valeur de pH inférieure à 6.

Conformément au procédé de préparation d'aldéhyde hydroxyaromatique selon l'invention, ladite solution saline d'hydroxyde, préférentiellement ladite solution d'hydroxyde de sodium, produite lors de ladite étape d) est avantageusement recyclée à ladite étape c) pour la mise en oeuvre de ladite réaction d'oxydation et/ou à ladite étape b) du procédé de séparation décrit ci-dessus et/ou à ladite étape de réaction de condensation en amont dudit procédé de séparation décrit ci-dessus.

Conformément au procédé de préparation d'aldéhyde hydroxyaromatique selon l'invention, lesdites membranes échangeuses de cations, capables d'assurer la séparation des cations présents dans le milieu aqueux basique issu de la réaction d'oxydation, et les membranes bipolaires, capables de neutraliser ledit milieu basique, permettent la neutralisation dudit composé hydroxylate d'alkoxybenzaldéhyde en aldéhyde hydroxyaromatique tout en limitant considérablement la production de sels, en particulier de sels de sulfate (généralement sous la forme de sels de sulfate de sodium), dès lors que l'acidification du milieu basique issu de ladite étape d'oxydation, opérée par électrodialyse (électro-électrodialyse et électrodialyse à membrane bipolaire), ne nécessite pas l'emploi d'un acide.

A l'issue de la mise en oeuvre de ladite étape d), l'aldéhyde hydroxyaromatique, préférentiellement la vanilline et/ou l'éthylvanilline, se trouve en solution aqueuse. Il est alors avantageusement extrait par un solvant organique qui solubilise l'aldéhyde hydroxyaromatique contenu dans la phase aqueuse, ledit solvant étant inerte par rapport à l'aldéhyde hydroxyaromatique.

Pour isoler l'aldéhyde hydroxyaromatique, notamment la vanilline et/ou l'éthylvanilline, du solvant d'extraction, on peut réaliser une séparation par recristallisation mais de préférence on effectue une distillation permettant d'obtenir par exemple en tête de distillation le solvant d'extraction (s'il est le composé le plus volatil du mélange), et par exemple en pied de distillation, un aldéhyde hydroxyaromatique, à savoir un mélange comprenant essentiellement ledit aldéhyde hydroxyaromatique, associé à des impuretés lourdes dénommées "goudrons" et à de faibles quantités d'impuretés légères. On fait référence pour cette étape en particulier au procédé décrit dans la demande de brevet WO 2010/007161.
Eventuellement ledit aldéhyde hydroxyaromatique peut être traité pour le conditionner sous forme solide. De préférence il est purifié en opérant par distillation suivie d'une cristallisation (par utilisation d'un ou plusieurs solvants ou par technique d'écaillage). Le produit résultant peut être éventuellement broyé pour obtenir des grains plus fins.

Les effluents du procédé selon l'invention peuvent être traités par les procédés classiques de traitement des effluents, comme cela est décrit par exemple dans la demande internationale de brevet WO 2013/135885.
Selon un mode de réalisation, un traitement d'oxydation peut être mis en oeuvre. Ce traitement d'oxydation peut requérir un agent oxydant, qui est de préférence H₂O₂, mais qui peut être un autre agent oxydant comme l'oxygène, ledit traitement d'oxydation étant de préférence réalisé en présence d'UV. On préfère réaliser ladite étape d'oxydation par un traitement d'oxydation avancée (AOP - Advanced Oxidation Process) en présence d'O₂ et/ou de H₂O₂ et d'UV ou un traitement dit « Fenton » comprenant l'oxydation des composés organiques en présence d'un agent oxydant et éventuellement de fer (II) et/ou fer (III).
Selon un autre mode de réalisation, les effluents peuvent être soumis à un biotraitement par mise en contact des composés organiques aromatiques en présence de bactéries ou enzymes dégradant lesdits composés organiques. Parmi les bactéries et enzymes utilisables ont peut citer sans être exhaustif : *Pseudomonas Putida, Pseudomonas mendocina, Pseudomonas putida, Comonas, Anthrobacter sp., Aspergillus niger,* mandelate dehydrogenase, benzoyldecarboxylase, vanillyl dehydrogenase, des toluène monooxygenase, catechol-1,2-dioxygenase, catechol-2,3-dioxygenase, la procatechuate-3,4-dioxygenase, procatechuate decarboxylate, etc.
Selon un autre mode de réalisation, le traitement des effluents peut comprendre étape d'adsorption des composés organiques aromatiques sur un substrat, comme par exemple du charbon actif, ou bien une ou plusieurs extractions liquide-liquide.
Ces variantes peuvent être combinées.
Toutefois, les effluents du procédé selon l'invention ayant avantageusement une teneur réduite voire nulle en sels de sulfate, le procédé de traitement des effluents peut être simplifié. L'utilisation de biotraitement uniquement est par exemple possible.

L'invention sera expliquée plus en détail au moyen de l'exemple ci-après d'un mode préféré de réalisation de l'invention, donné à titre non limitatif.

### EXEMPLE

Dans l'exemple, on définit le taux de conversion et la sélectivité obtenus.
Le taux de conversion ou taux de transformation (TT) correspond au rapport entre le nombre de moles de réactif transformé et le nombre de moles de réactif engagé.
La sélectivité ou le rendement par rapport au produit transformé (RT), est exprimé par le rapport entre le nombre de moles de produit formé et le nombre de moles de réactif transformé.

### Réaction de condensation

Dans un premier réacteur en inox 316L de 150 mL muni d'une double enveloppe, d'une agitation mécanique, d'une électrode de pH, d'un système réfrigérant et d'une arrivée de gaz inerte, on charge en continu :
- 1,14 kg/h d'eau déminéralisée
- 164 g/h (2,05 mol/h) d'une solution aqueuse de soude à 50% en poids.
- 178 g/h (1,44 mol/h) de gaïacol (neuf et recyclé).

Ce mélange réactionnel est maintenu à la température de 35°C. Cette préparante est ensuite alimentée dans le premier réacteur d'un système de 3 réacteurs en cascade avec une solution aqueuse d'acide glyoxylique à 50% en poids (107 g/h soit 0,72 mol/h). Les 3 réacteurs parfaitement agités sont en inox 316L et ont chacun un volume de 1,5L ; ils opèrent à 35°C.
Le temps de séjour global est de 2,1 heures.
A la sortie du dernier réacteur, on prélève un échantillon de ce milieu réactionnel et on dose par chromatographie en phase liquide les composés présents dans le mélange. Les résultats obtenus sont les suivants :
- conversion du gaïacol (GA) : TT = 47%
- sel disodique d'acide 4-hydroxy-3-méthoxy mandélique (APM(2Na)) :
   RT(APM(2Na)/GA)=86%
- sel disodique d'acide 2-hydroxy-3-méthoxy mandélique (AOM(2Na)) :
   RT(AOM(2Na)/GA)=6%
- sel trisodique d'acide 2-hydroxy-3-méthoxy 1,5-dimandélique (ADM(3Na)) :
   RT(ADM(3Na)/GA)=8%
Le milieu réactionnel est envoyé vers la section décantation / neutralisation / adsorption pour séparer le gaïacolate de sodium en excès des sels d'acides vanillyl-mandéliques.

### Décantation/Neutralisation

Le milieu de sortie de condensation est transféré par pompe à un débit de 1,6 kg/h dans un réacteur en inox 316L de 1 L, muni d'une agitation mécanique, d'une électrode de pH et d'une arrivée de CO₂ par une crépine immergée au fond du réacteur. Le débit de CO₂ est régulé pour que le pH du milieu réactionnel soit neutralisé à pH = 10,5.

Puis le milieu réactionnel est transféré dans un décanteur de 2L, muni d'une double-enveloppe, d'un système de maintien en température et dont le temps de séjour global est de 1,2 heure. La décantation a lieu à 35°C.

La décantation produit 2 flux :
- La couche organique, soutirée en fond de décanteur, comprenant du gaïacol, à un débit de 70 g/h. Ce flux est destiné à être recyclé directement à l'alimentation de la condensation.
- La couche aqueuse, phase légère, qui contient les sels d'acides vanillyl-mandéliques à oxyder et 2,9% pds de gaïacolate de sodium.

Pour optimiser les conditions de fonctionnement de la colonne d'adsorption, la phase aqueuse est à nouveau neutralisée à pH = 10,0 dans un réacteur en inox 316L de 1 L, muni d'une agitation mécanique, d'une électrode de pH et d'une arrivée de CO₂ par une crépine immergée au fond du réacteur. Le débit de CO₂ est régulé pour que le pH du milieu réactionnel soit neutralisé à pH = 10,0.

### Adsorption

L'adsorption du gaïacol est effectuée dans une colonne en verre, équipée d'une double-enveloppe et d'un système de maintien en température à 35°C. L'adsorbant mis en oeuvre est du charbon actif Norit ® C Gran du fournisseur Cabot Norit Activated Carbon : le lit de charbon actif dans la colonne a un volume de 2,0 L.

Les différentes étapes du cycle sont gérées par un automate qui met en marche et arrête les différentes pompes d'alimentation et de collecte par temporisation. Les temporisations ont été préalablement réglées pour alimenter des quantités massiques bien définies selon les étapes.

Le cycle d'adsorption comporte 4 étapes :

### - Adsorption :

Le milieu de sortie de condensation, décanté et neutralisé, alimente la tête de colonne d'adsorption par une pompe péristaltique à un débit de 3l/h. En sortie de colonne, le flux est dirigé vers le stockage tampon destiné à alimenter l'oxydation.
Après alimentation de 2,9 kg de milieu de condensation, l'automate active la deuxième étape du cycle.

### - Début du cycle de régénération :

La colonne est alimentée en tête avec de l'eau et de la soude 10% par une pompe péristaltique à un débit de 3 l/h. En pied de colonne, le flux de sortie continue d'être dirigé vers la réaction d'oxydation.
Lorsque 1,8 kg d'eau puis de NaOH 10% ont été alimentés vers la tête de colonne, l'automate active la troisième étape du cycle.

### - Régénération :

La colonne est alimentée en tête avec de l'eau par une pompe péristaltique à un débit de 3 l/h. En pied de colonne, le flux de sortie est dirigé vers le stockage tampon destiné au recyclage du gaïacolate de sodium en phase aqueuse vers la condensation.
Après alimentation de 1,4 kg d'eau vers la tête de colonne, l'automate active la quatrième étape du cycle.

### - Fin de cycle :

La colonne est alimentée en tête avec le milieu de sortie de condensation décanté et neutralisé, par une pompe péristaltique à un débit de 3 l/h. En pied de colonne, le flux est dirigé vers le stockage destiné au recyclage du gaïacolate de sodium en phase aqueuse vers la condensation.
Lorsque 1,0 kg de milieu de condensation a été alimenté vers la tête de colonne, l'automate active la première étape du cycle suivant.

Le débit vers l'oxydation, moyenné sur le cycle, est de 1,9 kg/h alors qu'il est de 1,0 kg/h pour la solution de gaïacolate de sodium recyclée à la condensation.

Dans chacun des 2 fûts de sortie de colonne, on prélève un échantillon et on dose par chromatographie en phase liquide les composés présents dans le mélange.

Les résultats obtenus sont les suivants :

### Milieu alimentant l'oxydation :

Gaïacolate de sodium (GANa) : 1200 ppm
Sel disodique d'APM (APM(2Na)) : 7,0% pds

### Gaïacolate de sodium recyclé à la condensation :

Gaïacolate de sodium (GANa) : 3,5% pds
Sel disodique d'APM (APM(2Na)) : 0,2% pds

Le taux de régénération du gaïacol adsorbé sur la colonne est de 100%.

### Réaction d'oxydation

Le réacteur d'oxydation en inox 316L équipé d'une agitation autoaspirante de type à cavitation ("cavitator") ou de type Rushton et d'une double enveloppe permettant un refroidissement efficace est alimenté en continu par :
- Le mélange du catalyseur et de la solution aqueuse de sels d'acides vanillyl-mandéliques issue de la colonne d'adsorption soit :
   ∘ 1,95 kg/h de milieu réactionnel de condensation dont le gaïacolate de sodium en excès a été séparé (puis recyclé) par adsorption sur la colonne de charbon actif. Ce mélange contient de l'ordre de 136 g/h de sel disodique d'acide 4-hydroxy-3-méthoxy mandélique, 9 g/h de sel disodique d'acide 2-hydroxy-3-méthoxy mandélique et 16 g/h de sel trisodique d'acide 2-hydroxy-3-méthoxy 1,5-dimandélique.
   ∘ 2 g/h d'une solution aqueuse de CuSO₄ en une quantité exprimée en pourcentage molaire de métal par rapport à la somme molaire des sels d'acides vanillyl-mandéliques : 0,04% chacun.
- la quantité adéquate d'une solution aqueuse de soude à 50% en poids correspondant au moins à la quantité requise par la stoechiométrie de la réaction d'oxydation.
- La quantité d'oxygène à pression atmosphérique suffisante pour avoir une conversion quasi complète des sels d'acides vanillyl-mandéliques. L'oxydant peut être de l'oxygène à pression atmosphérique ou de l'air sous pression.
Cette réaction s'effectue à 80°C.

A la sortie du réacteur, on prélève un échantillon de ce milieu réactionnel et on dose les composés présents dans le mélange par chromatographie en phase liquide.
Les résultats obtenus sont les suivants :
- Conversion du sel disodique de l'acide 4-hydroxy-3-méthoxy mandélique : TT > 99,5%
- Rendement en vanillate de sodium VANa :
   RT(VANa)/APM = 97%
La solution est stockée dans un bac avant d'être envoyée vers la section d'électrodialyse.

L'acidification de la solution de vanillate de sodium en sortie d'oxydation est réalisée en batch dans un pilote d'électrodialyse qui se compose d'un électrodialyseur, de trois circuits hydrauliques, dénommés respectivement électrolyte, sel/acide et base, comportant chacun une pompe de circulation et un débitmètre à flotteur (rotamètre). Les circuits sont maintenus à 53°C grâce à une circulation d'eau chaude, fournie par un bain thermostaté, dans des serpentins internes. L'électrodialyseur, d'une section de 0,02 m² comprend, à chaque extrémité un support sur lequel est fixé chaque électrode (anode et cathode en nickel), un empilement membranaire composé de 2 membranes d'extrémités cationiques de type Neosepta C6610, de 7 membranes bipolaires de type NEOSEPTA BP1 empilées par couches successives en alternance avec 7 membranes cationiques Neosepta CMB.
2448 g d'une solution de soude à 1 mol/l et 2032 g d'une solution de soude à 0,5 mol/l sont respectivement introduites dans les circuits « électrolyte » et « base ». Parallèlement, 2256 g d'une solution de vanillate de sodium contenant 4,1% de vanilline, 0,1% d'ortho vanilline, 0,6% de soude, 4% de carbonate de sodium, 0,5% d'acide vanillyl mandélique sont introduits dans le compartiment « sel/acide». Les mesures de pH et de conductivité sont assurées par des sondes plongeant dans ce compartiment. Les pompes sont mises en marche afin d'assurer la circulation des différentes solutions (300 l/h pour la solution d'électrolyte et 200 l/h pour les solutions « sel/acide » et « base ») et l'homogénéisation de la température. Le générateur de courant est ensuite allumé et la consigne d'intensité réglée à 15 A à l'aide du potentiomètre, soit une densité de courant de 0,75 kA/m². La diminution du pH est suivie au cours du temps. Un transfert des ions sodium s'opère de la solution « sel/acide » vers la solution « base ». Un dégagement important de CO₂ est observé à partir d'un pH voisin de 8, nécessitant un ajustement du débit de la solution de « sel/acide ». Lorsque le pH atteint 5,3 (soit après environ 50 minutes), le générateur de courant et les pompes de circulation sont arrêtées. Les solutions dans les trois compartiments sont vidangées, pesées et analysées. Les quantités récupérées sont respectivement 2448 g de solution d'électrolyte, 2288 g de solution de base et 1980 g de solution de sel/acide. Le rendement faradique obtenu, correspondant au rapport du nombre de moles de charges électriques élémentaires réellement transférés sur la quantité de charges électriques qui a traversé l'empilement, est estimé à environ 80%. Le taux de conversion du vanillate de sodium en vanilline est supérieur à 95%. Cette dernière est ensuite extraite en phase organique par ajout d'un solvant approprié.

## Revendications

1. Procédé de séparation d'au moins un composé mandélique sous forme salifiée à partir d'un milieu réactionnel aqueux résultant de la réaction de condensation, dans l'eau, d'au moins un composé aromatique porteur d'au moins un groupe hydroxyle et dont la position en para est libre avec l'acide glyoxylique en milieu basique, ledit procédé comprenant au moins les étapes suivantes :
a) La décantation dudit milieu réactionnel de manière à récupérer une phase organique comprenant essentiellement de l'excès dudit composé aromatique et une phase aqueuse formée d'au moins dudit composé mandélique et d'une quantité de l'excès dudit composé aromatique,
b) La mise en contact de ladite phase aqueuse avec un support adsorbant conduisant à l'adsorption sélective dudit composé aromatique sur ledit support et à la récupération d'un flux aqueux comprenant au moins ledit composé mandélique sous forme salifiée.

2. Procédé de séparation selon la revendication 1 dans lequel ledit composé aromatique est choisi parmi le phénol et les composés aromatiques hydroxylés répondant à la formule suivante (I) : 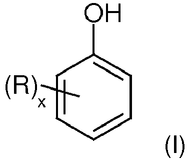 dans ladite formule,
- au moins la position en para du groupe hydroxyle est libre,
- R représente un atome d'hydrogène ou un ou plusieurs substituants, identiques ou différents,
- x, nombre de substituants sur un cycle, est un nombre inférieur ou égal à 4.

3. Procédé de séparation selon l'une des revendications 1 ou 2 dans lequel ledit composé aromatique est choisi parmi le phénol, l'o-crésol, le m-crésol, le 3-éthylphénol, le 2-tert-butylphénol, le gaïacol, le guétol.

4. Procédé de séparation selon l'une des revendications 1 à 3 dans lequel ladite phase organique obtenue après décantation est recyclée vers l'étape de réaction de condensation.

5. Procédé de séparation selon l'une des revendications 1 à 4 dans lequel ladite étape b) est opérée à co-courant.

6. Procédé de séparation selon l'une des revendications 1 à 5 dans lequel ledit flux aqueux obtenu à l'issue de ladite étape b) comprend un composé p-hydroxymandélique, un composé o-hydroxymandélique et un composé dimandélique hydroxylé.

7. Procédé de séparation selon l'une des revendications 1 à 6 dans lequel ledit composé aromatique, une fois désorbé, est recyclé vers l'étape de ladite réaction de condensation.

8. Procédé de séparation selon l'une des revendications 1 à 7 dans lequel on procède à une étape d'acidification dudit milieu réactionnel préalablement à ladite étape de décantation et/ou à une étape d'acidification de ladite phase aqueuse, issue de ladite étape de décantation, préalablement à ladite étape de mise en contact avec un support adsorbant.

9. Procédé de préparation d'aldéhyde hydroxyaromatique à partir de composé mandélique correspondant comprenant au moins les étapes suivantes :
a) La décantation d'un milieu réactionnel aqueux résultant de la réaction de condensation, dans l'eau, d'au moins un composé aromatique porteur d'au moins un groupe hydroxyle et dont la position en para est libre avec l'acide glyoxylique en milieu basique, de manière à récupérer une phase organique comprenant essentiellement de l'excès dudit composé aromatique et une phase aqueuse formée d'au moins dudit composé mandélique et d'une quantité de l'excès dudit composé aromatique,
b) La mise en contact de ladite phase aqueuse avec un support adsorbant conduisant à l'adsorption sélective dudit composé aromatique sur ledit support et à la récupération d'un flux aqueux comprenant au moins ledit composé mandélique sous forme salifiée
c) Une réaction d'oxydation d'au moins dudit composé mandélique en au moins un composé hydroxylate d'alkoxybenzaldéhyde,
d) La transformation d'au moins dudit composé hydroxylate d'alkoxybenzaldéhyde en aldéhyde hydroxyaromatique.

10. Procédé de préparation d'aldéhyde hydroxyaromatique selon la revendication 9 dans lequel ledit composé mandélique, soumis à ladite réaction d'oxydation, est un sel de l'acide p-hydroxymandélique, un sel de l'acide 4-hydroxy-3-méthoxymandélique, un sel de l'acide 3-éthoxy-4-hydroxymandélique, un sel de l'acide 4-hydroxy-3-isopropoxymandélique ou encore un mélange des sels des acides 4-hydroxy-3-méthoxymandélique et 3-éthoxy-4-hydroxymandélique.

11. Procédé de préparation d'aldéhyde hydroxyaromatique selon l'une des revendications 9 ou 10 dans lequel ledit composé hydroxylate d'alkoxybenzaldéhyde est un sel de l'acide 4-hydroxy-3-méthoxymandélique qui est transformé en vanilline à l'issue de ladite étape d).

12. Procédé de préparation d'aldéhyde hydroxyaromatique selon l'une des revendications 9 à 11 dans lequel ledit composé hydroxylate d'alkoxybenzaldéhyde est un sel de l'acide 3-éthoxy-4-hydroxymandélique qui est transformé en éthylvanilline à l'issue de ladite étape d).

13. Procédé de préparation d'aldéhyde hydroxyaromatique selon l'une des revendications 9 à 12 dans lequel ladite étape d) est mise en oeuvre par électro-électrodialyse au moyen de membranes échangeuses de cations.

14. Procédé de préparation d'aldéhyde hydroxyaromatique selon l'une des revendications 9 à 13 dans lequel ladite étape d) est opérée au moyen d'un électrolyseur à deux compartiments ou d'un électrolyseur à trois compartiments.

15. Procédé de préparation d'aldéhyde hydroxyaromatique selon l'une des revendications 9 à 14 dans lequel ladite étape d) est mise en oeuvre par électrodialyse à membrane bipolaire.

16. Procédé de préparation d'aldéhyde hydroxyaromatique selon la revendication 15 dans lequel ladite étape d) est opérée à une température comprise entre 45 et 90°C.

17. Procédé de préparation d'aldéhyde hydroxyaromatique selon l'une des revendications 9 à 16 dans lequel ladite solution saline d'hydroxyde, produite lors de ladite étape d), est recyclée à ladite étape c) pour la mise en oeuvre de ladite réaction d'oxydation et/ou à ladite étape b) du procédé de séparation selon l'une des revendications 1 à 8 et/ou à ladite étape de réaction de condensation en amont dudit procédé de séparation selon l'une des revendications 1 à 8.

## Patentansprüche

1. Verfahren zur Abtrennung von mindestens einer Mandelsäureverbindung in Salzform aus einem wässrigen Reaktionsmedium, das aus der Kondensationsreaktion, in Wasser, von mindestens einer aromatischen Verbindung, die mindestens eine Hydroxylgruppe trägt, deren para-Stellung frei ist, mit Glyoxylsäure in einem basischem Medium hervorgeht, wobei das Verfahren zumindest folgende Schritte umfasst:
a) Dekantieren des Reaktionsmediums zum Gewinnen einer organischen Phase, die im Wesentlichen den Überschuss der aromatischen Verbindung und eine wässrige Phase umfasst, die aus zumindest der Mandelsäureverbindung und einer Menge des Überschusses der aromatischen Verbindung gebildet ist,
b) Kontaktieren der wässrigen Phase mit einem adsorbierenden Träger, das zur gezielten Adsorption der aromatischen Verbindung an dem Träger und zur Gewinnung eines wässrigen Stroms führt, der zumindest die Mandelsäureverbindung in Salzform umfasst.

2. Verfahren zur Abtrennung nach Anspruch 1, wobei die aromatische Verbindung aus Phenol und den hydroxylierten aromatischen Verbindungen ausgewählt wird, die folgender Formel (I) entsprechen: 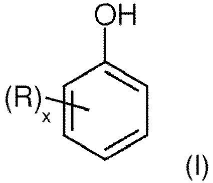 wobei in der Formel
- zumindest die para-Stellung der Hydroxylgruppe frei ist,
- R ein Wasserstoffatom oder ein oder mehrere identische oder unterschiedliche Substituenten darstellt,
- x, Anzahl von Substituenten in einem Ring, eine Zahl kleiner gleich 4 ist.

3. Verfahren zur Abtrennung nach Anspruch 1 oder 2, wobei die aromatische Verbindung aus Phenol, o-Kresol, m-Kresol, 3-Ethylphenol, 2-tert-Butylphenol, Guaiacol, Guethol ausgewählt wird.

4. Verfahren zur Abtrennung nach einem der Ansprüche 1 bis 3, wobei die organische Phase, die nach dem Dekantieren erhalten wird, zum Schritt der Kondensationsreaktion zurückgeführt wird.

5. Verfahren zur Abtrennung nach einem der Ansprüche 1 bis 4, wobei Schritt b) im Gegenstrom durchgeführt wird.

6. Verfahren zur Abtrennung nach einem der Ansprüche 1 bis 5, wobei der wässrige Strom, der am Ende von Schritt b) erhalten wird, eine p-Hydroxymandelsäureverbindung, eine o-Hydroxymandelsäureverbindung und eine hydroxylierte Dimandelsäureverbindung umfasst.

7. Verfahren zur Abtrennung nach einem der Ansprüche 1 bis 6, wobei die aromatische Verbindung, sobald sie desorbiert ist, zum Schritt der Kondensationsreaktion zurückgeführt wird.

8. Verfahren zur Abtrennung nach einem der Ansprüche 1 bis 7, wobei ein Schritt zur Ansäuerung des Reaktionsmediums vor dem Schritt des Dekantierens und/oder ein Schritt zur Ansäuerung der wässrigen Phase, die aus dem Dekantierschritt hervorgeht, vor dem Schritt des Kontaktierens mit einem adsorbierenden Träger durchgeführt wird.

9. Verfahren zur Herstellung von hydroxyaromatischem Aldehyd aus einer entsprechenden Mandelsäureverbindung, das zumindest folgende Schritte umfasst:
a) Dekantieren eines wässrigen Reaktionsmediums, das aus der Kondensationsreaktion, in Wasser, von mindestens einer aromatischen Verbindung, die mindestens eine Hydroxylgruppe trägt, deren para-Stellung frei ist, mit Glyoxylsäure in einem basischem Medium hervorgeht, zum Gewinnen einer organischen Phase, die im Wesentlichen den Überschuss der aromatischen Verbindung und eine wässrige Phase umfasst, die aus zumindest der Mandelsäureverbindung und einer Menge des Überschusses der aromatischen Verbindung gebildet ist,
b) Kontaktieren der wässrigen Phase mit einem adsorbierenden Träger, das zur gezielten Adsorption der aromatischen Verbindung an dem Träger und zur Gewinnung eines wässrigen Stroms führt, der zumindest die Mandelsäureverbindung in Salzform umfasst,
c) eine Oxidationsreaktion von zumindest der Mandelsäureverbindung zu mindestens einer Alkoxybenzaldehydhydroxylat-Verbindung,
d) Umsetzen von zumindest der Alkoxybenzaldehydhydroxylat-Verbindung zu hydroxyaromatischem Aldehyd.

10. Verfahren zur Herstellung von hydroxyaromatischem Aldehyd nach Anspruch 9, wobei die Mandelsäureverbindung, mit der die Oxidationsreaktion abläuft, ein Salz der p-Hydroxymandelsäure, ein Salz der 4-Hydroxy-3-methoxymandelsäure, ein Salz der 3-Ethoxy-4-hydroxymandelsäure, ein Salz der 4-Hydroxy-3-isopropoxymandelsäure oder auch eine Mischung aus Salzen der 4-Hydroxy-3-methoxymandelsäure und 3-Ethoxy-4-hydroxymandelsäure ist.

11. Verfahren zur Herstellung von hydroxyaromatischem Aldehyd nach Anspruch 9 oder 10, wobei die Alkoxybenzaldehydhydroxylat-Verbindung ein Salz der 4-Hydroxy-3-Methoxymandelsäure ist, das am Ende von Schritt d) zu Vanillin umgesetzt wird.

12. Verfahren zur Herstellung von hydroxyaromatischem Aldehyd nach einem der Ansprüche 9 bis 11, wobei die Alkoxybenzaldehydhydroxylat-Verbindung ein Salz der 3-Ethoxy-4-Hydroxymandelsäure ist, das am Ende von Schritt d) zu Ethylvanillin umgesetzt wird.

13. Verfahren zur Herstellung von hydroxyaromatischem Aldehyd nach einem der Ansprüche 9 bis 12, wobei Schritt d) mittels Kopplung von Elektrolyse und Elektrodialyse mithilfe von Kationenaustauschermembranen durchgeführt wird.

14. Verfahren zur Herstellung von hydroxyaromatischem Aldehyd nach einem der Ansprüche 9 bis 13, wobei Schritt d) mithilfe eines Zweikammer-Elektrolyseurs oder eines Dreikammer-Elektrolyseurs durchgeführt wird.

15. Verfahren zur Herstellung von hydroxyaromatischem Aldehyd nach einem der Ansprüche 9 bis 14, wobei Schritt d) mittels Elektrodialyse mit bipolarer Membran durchgeführt wird.

16. Verfahren zur Herstellung von hydroxyaromatischem Aldehyd nach Anspruch 15, wobei Schritt d) bei einer Temperatur zwischen 45 und 90 °C durchgeführt wird.

17. Verfahren zur Herstellung von hydroxyaromatischem Aldehyd nach einem der Ansprüche 9 bis 16, wobei die in Schritt d) hergestellte Salz-Hydroxidlösung zu Schritt c) zurückgeführt wird, damit die Oxidationsreaktion durchgeführt wird, und/oder zu Schritt b) des Verfahrens zur Abtrennung nach einem der Ansprüche 1 bis 8 und/oder zu dem Schritt der Kondensationsreaktion vor dem Verfahren zur Abtrennung nach einem der Ansprüche 1 bis 8.

## Claims

1. Process for separating at least one mandelic compound in salified form from an aqueous reaction medium resulting from the condensation reaction, in water, of at least one aromatic compound bearing at least one hydroxyl group and in which the para position is free with glyoxylic acid in basic medium, said process comprising at least the following steps:
a) decantation of said reaction medium so as to recover an organic phase essentially comprising the excess of said aromatic compound and an aqueous phase formed from at least said mandelic compound and an amount of the excess of said aromatic compound,
b) placing said aqueous phase in contact with an adsorbent support, leading to selective adsorption of said aromatic compound on said support and to the recovery of an aqueous stream comprising at least said mandelic compound in salified form.

2. Separation process according to Claim 1, in which said aromatic compound is chosen from phenol and the hydroxylated aromatic compounds corresponding to formula (I) below: 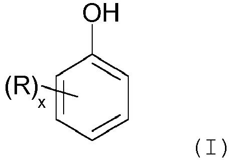 in said formula:
- at least the position para to the hydroxyl group is free,
- R represents a hydrogen atom or one or more identical or different substituents,
- x, the number of substituents on a ring, is a number less than or equal to 4.

3. Separation process according to either of Claims 1 and 2, in which said aromatic compound is chosen from phenol, o-cresol, m-cresol, 3-ethylphenol, 2-tert-butylphenol, guaiacol and guaethol.

4. Separation process according to one of Claims 1 to 3, in which said organic phase obtained after decantation is recycled into the condensation reaction step.

5. Separation process according to one of Claims 1 to 4, in which said step b) is performed co-currentwise.

6. Separation process according to one of Claims 1 to 5, in which said aqueous stream obtained after said step b) comprises a p-hydroxymandelic compound, an o-hydroxymandelic compound and a hydroxylated dimandelic compound.

7. Separation process according to one of Claims 1 to 6, in which said aromatic compound, once desorbed, is recycled into said condensation reaction step.

8. Separation process according to one of Claims 1 to 7, in which a step of acidification of said reaction medium is performed prior to said decantation step and/or a step of acidification of said aqueous phase, derived from said decantation step, is performed prior to said step of placing in contact with an adsorbent support.

9. Process for preparing hydroxyaromatic aldehyde from corresponding mandelic compound, comprising at least the following steps:
a) decantation of an aqueous reaction medium resulting from the condensation reaction, in water, of at least one aromatic compound bearing at least one hydroxyl group and in which the para position is free with glyoxylic acid in basic medium, so as to recover an organic phase essentially comprising the excess of said aromatic compound and an aqueous phase formed from at least said mandelic compound and an amount of the excess of said aromatic compound,
b) placing said aqueous phase in contact with an adsorbent support, leading to selective adsorption of said aromatic compound on said support and to the recovery of an aqueous stream comprising at least said mandelic compound in salified form,
c) an oxidation reaction of at least said mandelic compound, to at least one alkoxybenzaldehyde hydroxylate compound,
d) transformation of at least said alkoxybenzaldehyde hydroxylate compound into hydroxyaromatic aldehyde.

10. Process for preparing hydroxyaromatic aldehyde according to Claim 9, in which said mandelic compound, subjected to said oxidation reaction, is a p-hydroxymandelic acid salt, a 4-hydroxy-3-methoxymandelic acid salt, a 3-ethoxy-4-hydroxymandelic acid salt, a 4-hydroxy-3-isopropoxymandelic acid salt or a mixture of 4-hydroxy-3-methoxymandelic and 3-ethoxy-4-hydroxymandelic acid salts.

11. Process for preparing hydroxyaromatic aldehyde according to either of Claims 9 and 10, in which said alkoxybenzaldehyde hydroxylate compound is a 4-hydroxy-3-methoxymandelic acid salt which is converted into vanillin on conclusion of said step d).

12. Process for preparing hydroxyaromatic aldehyde according to one of Claims 9 to 11, in which said alkoxybenzaldehyde hydroxylate compound is a 3-ethoxy-4-hydroxymandelic acid salt which is converted into ethylvanillin on conclusion of said step d).

13. Process for preparing hydroxyaromatic aldehyde according to one of Claims 9 to 12, in which said step d) is performed by electro-electrodialysis using cation-exchange membranes.

14. Process for preparing hydroxyaromatic aldehyde according to one of Claims 9 to 13, in which said step d) is performed by means of a two-compartment electrolyzer or a three-compartment electrolyzer.

15. Process for preparing hydroxyaromatic aldehyde according to one of Claims 9 to 14, in which said step d) is performed by bipolar membrane electrodialysis.

16. Process for preparing hydroxyaromatic aldehyde according to Claim 15, in which said step d) is conducted at a temperature of between 45 and 90°C.

17. Process for preparing hydroxyaromatic aldehyde according to one of Claims 9 to 16, in which said saline hydroxide solution, produced during said step d), is recycled into said step c) to perform said oxidation reaction and/or into said step b) of the separation process according to one of Claims 1 to 8 and/or into said condensation reaction step upstream of said separation process according to one of Claims 1 to 8.
